# EUROPEAN PATENT APPLICATION

(11) **EP 4 194 544 A1**
(43) Date of publication of application: **14.06.2023**
(21) Application number: 20946455.1
(22) Date of filing: 17.08.2020
(51) Int. Cl.: C12N 1/21, C12P 19/26

(54) **STRAIN FOR PRODUCING N-ACETYLGLUCOSAMINE, AND CONSTRUCTION METHOD THEREFOR AND USE THEREOF**

(30) Priority: 24.07.2020 CN 202010725842
(71) Applicant: Shanghai Jiao Tong University, Shanghai 200240 (CN); Anhui Jointfree Bio-Scitech Co., Ltd., Bengbu, Anhui 233700 (CN)
(72) Inventor: XU, Ping, Shanghai 200240 (CN); WU, Yutong, Shanghai 200240 (CN); TAO, Fei, Shanghai 200240 (CN); SHANG, Haitao, Bengbu, Anhui 233700 (CN); MU, Xiaoling, Bengbu, Anhui 233700 (CN); ZHANG, Yi, Bengbu, Anhui 233700 (CN); DUAN, Zhongyue, Bengbu, Anhui 233700 (CN)
(74) Representative: Mewburn Ellis LLP
(86) International application number: PCT/CN2020/109480
(87) International publication number: WO 2022/016641

(57) **Abstract**

A genetically engineered bacterial strain that produces N-acetylglucosamine, as well as a method of construction and use thereof. The genetically engineered bacterial strain can ferment N-acetylglucosamine under a condition of 40-50°C. Through knocking out genes for glucosamine 6-phosphate deaminase, N-acetylglucosamine-6-phosphate deacetylase and the N-acetylglucosamine transporter protein from a parental bacterium, an N-acetylglucosamine catabolism pathway is blocked. Moreover, overexpression genes for glucosamine 6-phosphate synthase and glucosamine 6-phosphate acetylase are introduced, enabling extracellular accumulation of N-acetylglucosamine and high-temperature fermentation of N-acetylglucosamine at a temperature higher than 40 °C.

## Description

### Field of the Invention

The present invention pertains to the field of biological engineering and relates to a genetically engineered strain that produces N-acetylglucosamine as well as to a method of construction and use thereof.

### Description of the Prior Art

N-acetylglucosamine (GlcNAc), a building block of various polysaccharides in organisms, is formed by replacing a hydroxyl group in glucose with an amino group, particularly rich in the exoskeleton of crustaceans and extensively used in the food, pharmaceutical and cosmetic industries. In the food industry, it can be used as a food antioxidant, an additive for food for infants and young children and a sweetener for diabetics. In the pharmaceutical industry, as a novel biochemical drug, it is clinically used to treat rheumatic and rheumatoid arthritis. Another important use is to enhance the functionality of the human immune system, suppress excessive growth of cancer cells or fibroblasts, and inhibit or treat cancer and malignant tumors. In addition, N-acetylglucosamine also has a therapeutic effect on osteoarthritis and joint pain. In the cosmetic industry, it can form with D-glucuronic acid macromolecular mucopolysaccharides for producing hyaluronic acid. This use is expected to have broad market prospects.

N-acetylglucosamine is produced primarily by chemical, enzymatic and microbial fermentation approaches. The chemical approach is to obtain N-acetylglucosamine from acid hydrolysis of chitin. It involves extracting chitin from crab and shrimp shells and then producing glucosamine by acid hydrolysis. Subsequently, N-acetylglucosamine is hydrolyzed and deacetylated by concentrated hydrochloric acid directly into glucosamine. Acetic anhydride can be used to acetylate glucosamine into N-acetylglucosamine. However, this approach is not environmentally friendly and highly polluting and precludes patients with seafood allergies. Compared with the chemical approach, both the enzymatic and microbial fermentation approaches are environmentally friendly. However, the enzymatic approach involves the use of enzymes for catalyzing the degradation of chitin and the synthesis of N-acetylglucosamine, and limited by high pre-treatment complexity of shrimp and crab shells as the substrate, low activity of predominant enzymes and difficulties in product separation and purification, it is very challenging to put it into mass production. Microbial fermentation is currently the most promising method for producing N-acetylglucosamine and is also a focus of research in the art to which the invention pertains.

Compared with low-temperature fermentation at 40 °C or below, high-temperature fermentation has the advantages of minimizing the risk of contamination, speeding up the conversion of raw materials, reducing the cost of heat exchange, etc. All the reports so far on production of N-acetylglucosamine by metabolically engineered microorganisms have utilized fermentation temperatures of 38 °C and lower, there is still no patent or report regarding high-temperature production of N-acetylglucosamine. Therefore, there is a need for developing a low-cost, robust microbial platform capable of high-temperature, high-yield production of N-acetylglucosamine. Thermophiles such as *Bacillus coagulans, Bacillus licheniformis* and *Bacillus stearothermophiluscan* grow under high-temperature conditions (40 °C and above) and utilize organic carbon sources (glucose, xylose, arabinose, etc.) for fermentation. For example, *Bacillus licheniformis (B. licheniformis*) ATCC 14580 is a facultative anaerobic, Gram-positive, endospore-forming bacterium, which can utilize various five- and six-carbon sugars and requires a shorter fermentation cycle because of a fast cell growth rate. It allows for stable genetic manipulation and has been recognized by the US Food and Drug Administration as a "generally regarded as safe" (GRAS) strain. Moreover, this strain can ferment at a temperature as high as 50 °C. These advantages suggest that ATCC 14580 can be used as an ideal platform strain.

### Summary of the Invention

In order to overcome the above-described problems, the present invention provides a genetically engineered strain capable of producing N-acetylglucosamine under high-temperature conditions, as well as a method of construction and use thereof. Taking a thermophilic *B. licheniformis* strain as an example, according to the present invention, an N-acetylglucosamine catabolism pathway is eliminated from, and an N-acetylglucosamine-producing metabolism pathway is introduced into, the *B*. *licheniformis* strain, enabling extracellular accumulation of N-acetylglucosamine and efficient production of N-acetylglucosamine under a high-temperature condition. The strain construction concept proposed in the present invention can be utilized to develop strains for high-temperature production of various products. The proposed strain and fermentation process have good application prospects in industrial high-temperature N-acetylglucosamine fermentation.

In one aspect of the present invention, there is provided a genetically engineered strain that produces N-acetylglucosamine, which can ferment N-acetylglucosamine under a condition of 40-50 °C.

Additionally, an original strain of the genetically engineered strain is thermophilus.

Additionally, an original strain of the genetically engineered strain is *bacillus.*

Preferably, an original strain of the genetically engineered strain is *Bacillus licheniformis, Bacillus coagulans, Bacillus methylotrophicus,* thermophilic *Bacillus inulinus, Geobacillus stearothermophilus* or the like.

Preferably, an original strain of the genetically engineered strain is *Bacillus licheniformis* ATCC 14580, which is available for direct purchase from ATCC's website.

Additionally, the genetically engineered strain is *Bacillus licheniformis* BNGS1, deposited in China Center for Type Culture Collection on March 14, 2020 as CCTCC NO. M2020054.

Additionally, a catabolism pathway and an intracellular transport pathway of N-acetylglucosamine and N-acetylglucosamine intermediates in an original strain of the genetically engineered strain are blocked.

Preferably, the catabolism pathway for N-acetylglucosamine and N-acetylglucosamine intermediates is blocked by deactivation or deletion of one or more of the *nagB* and *gamA* genes for glucosamine 6-phosphate deaminase and the *nagA* gene for N-acetylglucosamine-6-phosphate deacetylase and the intracellular transport pathway for N-acetylglucosamine is blocked by deactivation or deletion of one or both of the *gamP* and *nagP* genes for the N-acetylglucosamine transporter protein.

Additionally, over-expressing genes for glucosamine 6-phosphate synthase and glucosamine 6-phosphate acetylase are introduced into the genetically engineered strain.

Additionally, a sequence of the gene for glucosamine 6-phosphate acetylase is as shown in SEQ ID NO. 1, and a sequence of the gene for glucosamine 6-phosphate synthase is as shown in SEQ ID NO. 2.

Preferably, the gene for glucosamine 6-phosphate synthase can be obtained from PCR amplification using the DNA sequence of the whole genome of *B. licheniformis* (GenBank No. NC_006270.3) as a template.

Preferably, the gene for glucosamine 6-phosphate acetylase can be obtained by whole gene synthesis with codon optimization according to the whole genome of *Saccharomyces cerevisiae* (GenBank No. NM_001179949).

In another aspect of the present invention, there is provided a method of constructing the genetically engineered strain as defined above. In one specific embodiment, the method includes: deactivating or deleting one or more of the genes for glucosamine 6-phosphate deaminase, N-acetylglucosamine-6-phosphate deacetylase and the N-acetylglucosamine transporter protein in an N-acetylglucosamine catabolism pathway of an original strain; and introducing over-expressing genes for glucosamine 6-phosphate synthase and glucosamine 6-phosphate acetylase.

Additionally, the method includes the steps of:
A. obtaining a knockout strain by knocking out the *nagB* and *gamA* genes for glucosamine 6-phosphate deaminase, the *nagA* gene for N-acetylglucosamine-6-phosphate deacetylase and the *gamP* and *nagP* genes for the N-acetylglucosamine transporter protein of the original strain;
B. constructing a double-expression vector containing both the *glmS gene* for glucosamine 6-phosphate synthase and the *GNA1* gene for glucosamine 6-phosphate acetylase; and
C. obtaining the genetically engineered strain that produces N-acetylglucosamine by transferring the double-expression vector into the knockout strain obtained in step A.

Additionally, the double-expression vector is constructed by introducing promoters and expressing the expression vector in series with the promoters and the *glmS* and *GNAl* genes.

Preferably, the expression vector is pHY300PLK.

Preferably, the promoters are Pₐₗₛ, P₄₃, Pₛₜ and Pₐₚᵣₑ.

Preferably, a sequence of the Pₐₗₛ promoter is as shown in SEQ ID NO. 3, a sequence of the P₄₃ promoter is as shown in SEQ ID NO. 4, a sequence of the Pₛₜ promoter is as shown in SEQ ID NO. 5, and a sequence of the Pₐₚᵣₑ promoter is as shown in SEQ ID NO. 6.

Preferably, the *glmS* gene for glucosamine 6-phosphate synthase originates from *Bacillus licheniformis, Bacillus coagulans, Bacillus methylotrophicus,* thermophilic *Bacillus inulinus, Geobacillus stearothermophilus* or the like.

Preferably, the *GNA1* gene for glucosamine 6-phosphate acetylase originates from *Saccharomyces cerevisiae* or another microorganism that produces a thermophilic enzyme with the same functionality, such as *Kluyveromyces marxianus, Nadsonia fulvescens* or the like.

Preferably, the original strain is *Bacillus licheniformis* ATCC 14580.

In a further aspect of the present invention, there is provided use of the genetically engineered strain as defined above, in particular for production of N-acetylglucosamine.

Additionally, a fermentation temperature of the production is 25 °C to 50 °C.

Preferably, a fermentation temperature of the production is 40 °C to 50 °C.

Additionally, a carbon source utilized in the production is glucose, glycerol, xylose, arabinose or the like.

Additionally, a seed culture is obtained from seed cultivation of the genetically engineered strain, followed by secondary activation in a fermentation medium using glucose as a carbon source. Finally, it is transferred into a fermenter for fermentation of N-acetylglucosamine. Specifically, this includes the steps of:
1) seed cultivation: inoculating the genetically engineered strain into a seed medium, adding tetracycline resistance thereto and culturing it for 12-16 h at 50 °C and 200 rpm for seed activation;
2) shake flask cultivation: transferring the activated seed in step 1) at an inoculation rate of 5% to a shake flask containing a fermentation medium, adding tetracycline resistance thereto and culturing it for 12-16 h at 50 °C and 200 rpm for secondary activation; and
3) fermentation cultivation: transferring it to a fermenter at an inoculation rate of 4%, adding tetracycline resistance and a carbon source, performing fed-batch fermentation at 40 °C to 50 °C, and culturing it until the end of fermentation.

Preferably, the seed medium in step 1) comprises following components: peptone, yeast powder and sodium chloride. Preferably, it contains 10 g/l of peptone, 5 g/l of yeast powder and 10 g/l of sodium chloride.

Preferably, the fermentation medium in step 2) comprises following components: yeast powder, peptone, ammonium sulfate, dipotassium hydrogen phosphate trihydrate, potassium dihydrogen phosphate and glucose. Preferably, it contains 12 g/l of yeast powder, 6 g/l of peptone, 6 g/l of ammonium sulfate, 18.75 g/l of dipotassium hydrogen phosphate trihydrate, 2.5 g/l of potassium dihydrogen phosphate and 30 g/l of glucose.

Preferably, in step 2), the shake flask is a 500-ml Erlenmeyer flask, and the fermentation medium is contained at an amount of 75 ml.

Preferably, the fermentation medium in step 3) comprises following components: yeast powder, corn steep liquor powder, ammonium sulfate, dipotassium hydrogen phosphate trihydrate, potassium dihydrogen phosphate and glucose. Preferably, it contains 12 g/l of yeast powder, 6 g/l of corn steep liquor powder, 6 g/l of ammonium sulfate, 18.75 g/l of dipotassium hydrogen phosphate trihydrate, 2.5 g/l of potassium dihydrogen phosphate and 30 g/l of glucose.

Preferably, fermentation conditions in step 3) are: a pH value maintained at 7.0 using a 3 mol/l hydrochloric acid solution and a 25% (v/v) ammonia solution; a ventilation rate of 1.5 vvm; an initial agitation rate of 600 rpm; a glucose concentration continuously controlled at 30 g/l by supplementing glucose upon it being consumed to 3-4 g/l.

The genetically engineered strain provided in the present application enables high-temperature production of N-acetylglucosamine. Moreover, during fermentation of the genetically engineered strain, the addition of an inducer is dispensed with because of constitutive expression. In addition, the fermentation process seldom requires temperature reduction, and fermentation is allowed to proceed at a significantly increased rate at the high temperature. Further, since the high temperature inhibits the growth of other microorganisms, open fermentation is possible, which can effectively reduce the production cost. The present invention enables fermentation of N-acetylglucosamine at a relatively high temperature of 40 °C to 50 °C and has high value in industrial applications. The strain construction concept proposed in the present invention can be utilized to develop strains for high-temperature production of various products. The proposed strain and fermentation process have good application prospects in industrial high-temperature N-acetylglucosamine fermentation.

### Brief Description of the Drawing

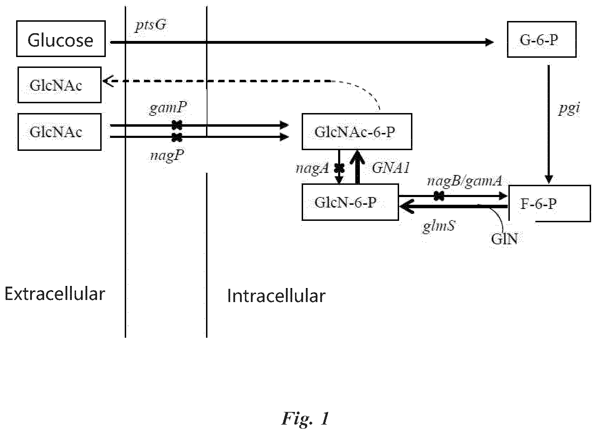

Fig. 1 shows an N-acetylglucosamine synthesis and metabolism pathway in an engineered *B. licheniformis* strain.

### Detailed Description of the Preferred Embodiments

An N-acetylglucosamine synthesis and metabolism pathway of an engineered strain described herein is as shown in Fig. 1, in which glutamine (Gln) serves as an amino acid donor, and glucosamine synthase encoded by the glmS gene converts fructose 6-phosphate (F-6-P) into glucosamine 6-phosphate (GlcN-6-P), which is in turn converted into N-acetyl-glucosamine 6-phosphate (GlcNAc-6-P) under the action of glucosamine 6-phosphate acetylase (*GNA1*). GlcNAc-6-P is dephosphorylated into N-acetylglucosamine under the catalysis of phosphorylase, which is then secreted to the outside of cells. Since the *nagB* and *gamA* genes that encode glucosamine 6-phosphate deaminase and the *nagA* gene that encodes N-acetylglucosamine-6-phosphate deacetylase are knocked out so as to be deactivated or so that the precursor of N-acetylglucosamine is less consumed in cells. Moreover, since the *gamP* and *nagP* genes encoding the N-acetylglucosamine transporter protein are knocked out, N-acetylglucosamine produced by the engineered strain cannot be transported and will accumulate to a high concentration outside cells.

According to the present application, a metabolism pathway for high-temperature fermentation of N-acetylglucosamine is established in a thermophilic *B. licheniformis* strain, in which the expression of a gene encoding a rate-limiting enzyme involved in N-acetylglucosamine synthesis is enhanced, and genes that may cause consumption and back diffusion of N-acetylglucosamine are deactivated or knocked out. Inhibiting consumption and back diffusion of N-acetylglucosamine enables N-acetylglucosamine produced by the engineered strain to accumulate to a high concentration.

In one aspect of the present invention, there is provided a genetically engineered strain that produces N-acetylglucosamine. This genetically engineered strain can ferment N-acetylglucosamine at 40-50 °C.

In one specific embodiment, an N-acetylglucosamine catabolism pathway in an original strain of the genetically engineered strain is blocked.

In another specific embodiment, the N-acetylglucosamine catabolism pathway is blocked as a result of deactivation or loss of one or more of the *nagB* and *gamA* genes that encode glucosamine 6-phosphate deaminase, the *nagA* gene that decodes N-acetylglucosamine-6-phosphate deacetylase and the *gamP* and *nagP* genes that encode the N-acetylglucosamine transporter protein.

As used herein, the term "blocking" refers to blocking a pathway by various genetic engineering approaches, including making the pathway unable to carry on by deactivating or deleting genes encoding one or more catalytic enzymes necessary for the pathway.

Those skilled in the art would appreciate that it is also impossible that the original strain does not contain the deleted or deactivated genes, such as those of glucosamine 6-phosphate deaminase, N-acetylglucosamine-6-phosphate deacetylase and the N-acetylglucosamine transporter protein, or even the blocked pathway. In this case, due to absence of the genes or pathway, the construction of the genetically engineered strain would not involve the deactivation, deletion or blocking achieved by genetic engineering.

The present invention will be further described below by way of specific examples.

The following examples are illustrative and do not limit the scope of protection of the present invention in any sense. Experimental methods to which reference is made in the following description of the examples are all conventional methods, unless otherwise noted. Materials, reagents and the like to which reference is made in the following description of the examples are obtainable from commercial sources, unless otherwise noted.

### Example 1: Knockout of B. licheniformis nagP Gene

1. Primers were designed according to the sequence of the *B. licheniformis* MW3 genome (GenBank No. NC_006270.3): nagP-U-F (upstream primer, GGTACCCGGGAGCTCATGAATGAGGAGGATCACACAGTC, SEQ ID NO. 7) and nagP-U-R (downstream primer, GAAGGGGCTTATCTTAGTTAAAACCCCTTTCGATGATATT, SEQ ID NO. 8). An 800-bp fragment upstream of the *nagP* gene was obtained by PCR amplification using the DNA of the *B. licheniformis* MW3 genome as a template.
2. Primers were designed according to the sequence of the *B. licheniformis* MW3 genome (GenBank No. NC_006270.3): nagP-D-F (upstream primer, AAAGGGGTTTTAACTAAGATAAGCCCCTTCTGAGGAAG, SEQ ID NO. 9) and nagP-D-R (downstream primer, GCGTCGGGCGATATCGAGGCGGACGAATACTTTGAC, SEQ ID NO. 10). An 800-bp fragment downstream of the *nagP* gene was obtained by PCR amplification using the DNA of the *B. licheniformis* MW3 genome as a template.
3. After the two PCR products were purified, 1 µl of each was taken as a template and inserted into a pKVM vector using a seamless cloning technique. As a result, a knockout pKVM vector-Δ*nagP* was obtained.
4. The knockout pKVM vector-Δ*nagP* was transformed into *Escherichia coli* S17-1 for conjugative transfer with *B. licheniformis* and for knockout of the *nagP* gene by homologous recombination. The primers used were nagP-U-F (GGTACCCGGGAGCTCATGAATGAGGAGGATCACACAGTC, SEQ ID NO. 7) and nagP-D-R (GCGTCGGGCGATATCGAGGCGGACGAATACTTTGAC, SEQ ID NO. 10). Successful amplification of a 1600-bp fragment was considered as a sign of successful screening of a positive double crossover clone as well as of successful knockout of the gene. Finally, a knockout strain MW3△*nagP* was obtained.

### Example 2: Additional Knockout of B. licheniformis gamP Gene

1. Primers were designed according to the sequence of the *B. licheniformis* MW3 genome (GenBank No. NC_006270.3): gamP-U-F (upstream primer, GGTACCCGGGAGCTCTAGGGTAAAACCGTATGCCGC, SEQ ID NO. 11) and gamP-U-R (downstream primer, AAGCAACTTCAGTTTTCCGGCATTCTCCTTATGTCAA, SEQ ID NO. 12). An 800-bp fragment upstream of the *gamP* gene was obtained by PCR amplification using the DNA of the *B. licheniformis* MW3 genome as a template.
2. Primers were designed according to the sequence of the *B. licheniformis* MW3 genome (GenBank No. NC_006270.3): gamP-D-F (upstream primer, AAGGAGAATGCCGGAAAACTGAAGTTGCTTTTGAGGAATC, SEQ ID NO. 13) and gamP-D-R (downstream primer, GCGTCGGGCGATATCGGAAATTTCTCTGCCAGCTGC, SEQ ID NO. 14). An 800-bp fragment downstream of the *gamP* gene was obtained by PCR amplification using the DNA of the *B. licheniformis* MW3 genome as a template.
3. After the two PCR products were purified, 1 µl of each was taken as a template and inserted into a pKVM vector using a seamless cloning technique. As a result, a knockout pKVM vector-△*gamP* was obtained.
4. The knockout pKVM vector*-*△*gamP* was transformed into *Escherichia coli* S17-1 for conjugative transfer with the knockout *B. licheniformis* strain MW3△*nagP* and for knockout of the *gamP* gene by homologous recombination. The primers used were gamP-U-F (GGTACCCGGGAGCTCTAGGGTAAAACCGTATGCCGC, SEQ ID NO. 11) and gamP-D-R (GCGTCGGGCGATATCGGAAATTTCTCTGCCAGCTGC, SEQ ID NO. 14). Successful amplification of a 1600-bp fragment was considered as a sign of successful screening of a positive double crossover clone as well as of successful knockout of the gene. Finally, a knockout strain MW3△*nagP*△*gamP* was obtained.

### Example 3: Additional Knockout of B. licheniformis gamA Gene

1. Primers were designed according to the sequence of the *B. licheniformis* MW3 genome (GenBank No. NC_006270.3): gamA-U-F (upstream primer, GGTACCCGGGAGCTCGGTCAAGAGGGAGGGTTCACTT, SEQ ID NO. 15) and gamA-U-R (downstream primer, TGTCAGTCATTCAATGTTTTTCTCCTTTCCACAAAATAAA, SEQ ID NO. 16). An 800-bp fragment upstream of the *gamA* gene was obtained by PCR amplification using the DNA of the *B. licheniformis* MW3 genome as a template.
2. Primers were designed according to the sequence of the *B. licheniformis* MW3 genome (GenBank No. NC_006270.3): gamA-D-F (upstream primer, GGAAAGGAGAAAAACATTGAATGACTGACAAAATCGGTTA, SEQ ID NO. 17) and gamA-D-R (downstream primer, GCGTCGGGCGATATCTCATATCGGGGATCGGCTT, SEQ ID NO. 18). An 800-bp fragment downstream of the *gamA* gene was obtained by PCR amplification using the DNA of the *B. licheniformis* MW3 genome as a template.
3. After the two PCR products were purified, 1 µl of each was taken as a template and inserted into a pKVM vector using a seamless cloning technique. As a result, a knockout pKVM vector-△*gamA* was obtained.
4. The knockout pKVM vector-△*gamA* was transformed into *Escherichia coli* S17-1 for conjugative transfer with the knockout *B. licheniformis* strain MW3△*nagP*△*gamP* and for knockout of the *gamA* gene by homologous recombination. The primers used were gamA-U-F (GGTACCCGGGAGCTCGGTCAAGAGGGAGGGTTCACTT, SEQ ID NO. 15) and gamA-D-R (GCGTCGGGCGATATCTCATATCGGGGATCGGCTT, SEQ ID NO. 18). Successful amplification of a 1600-bp fragment was considered as a sign of successful screening of a positive double crossover clone as well as of successful knockout of the gene. Finally, a knockout strain MW3△*nag*P△*gamP*△*gamA* was obtained.

### Example 4: Additional Knockout of B. licheniformis nagAB Gene Cluster

1. Primers were designed according to the sequence of the *B. licheniformis* MW3 genome (GenBank No. NC_006270.3): nagAB-U-F (upstream primer, GGTACCCGGGAGCTCCCGCACGGTCAGCTTA, SEQ ID NO. 19) and nagAB-U-R (downstream primer, GGGAATCTTTTTTGATACAACTCTAGTTGTCTAGACCAAT, SEQ ID NO. 20). An 800-bp fragment upstream of the *nagAB* gene cluster was obtained by PCR amplification using the DNA of the *B. licheniformis* MW3 genome as a template.
2. Primers were designed according to the sequence of the *B. licheniformis* MW3 genome (GenBank No. NC_006270.3): nagAB-D-F (upstream primer, ACAACTAGAGTTGTATCAAAAAAGATTCCCACATT, SEQ ID NO. 21) and nagAB-D-R (downstream primer, GCGTCGGGCGATATCCCTCTTCATATCAATGACGAA, SEQ ID NO. 22). An 800-bp fragment downstream of the *nagAB* gene cluster was obtained by PCR amplification using the DNA of the *B. licheniformis* MW3 genome as a template.
3. After the two PCR products were purified, 1 µl of each was taken as a template and inserted into a pKVM vector using a seamless cloning technique. As a result, a knockout pKVM vector-△*nagAB* was obtained.
4. The knockout pKVM vector-△*nagAB* was transformed into *Escherichia coli* S17-1 for conjugative transfer with the knockout *B. licheniformis* strain MW3Δ*nagP*△*gamP*△*gamA* and for knockout of the *nagAB* gene cluster by homologous recombination. The primers used were nagAB-U-F (GGTACCCGGGAGCTCCCGCACGGTCAGCTTA, SEQ ID NO. 19) and nagAB-D-R (GCGTCGGGCGATATCCCTCTTCATATCAATGACGAA, SEQ ID NO. 22). Successful amplification of a 1600-bp fragment was considered as a sign of successful screening of a positive double crossover clone as well as successful knockout of the gene. Finally, a knockout strain MW3△*nagP*△*gamP*△*gamA*△*nagAB* was obtained.

### Example 5: Construction of GNA1-glmS Double Expression Vector

Primers were designed according to the sequence of the *B. licheniformis* MW3 genome (GenBank No. NC_006270.3): Pₐₗₛ-F (upstream primer, TATCGATAAGCTTGATATCGAAGGTGACGCCTATTTCACT, SEQ ID NO. 23) and Pₐₗₛ-R (downstream primer, GTCCGGCAGGCTCATAGCCCTCACTCCTCCATT, SEQ ID NO. 24). The Pₐₗₛ promoter sequence was obtained by PCR amplification using the DNA of the B. *licheniformis* MW3 genome as a template.

Whole gene synthesis was carried out using the P₄₃ promoter. The primers used were designed as P₄₃-F (upstream primer, TATCGATAAGCTTGATATCGTGTCGACGTGCATGCAG, SEQ ID NO. 25) and P₄₃-R (downstream primer, GTCCGGCAGGCTCATAGCCCTCACTCCTCCTATAAT, SEQ ID NO. 26). Moreover, the P₄₃ promoter sequence was obtained through PCR amplification.

Whole gene synthesis was carried out using the Pₛₜ promoter. The primers used were designed as Pₛₜ-F (upstream primer, TATCGATAAGCTTGATATCGCATGATGTGGGCGTTTTT, SEQ ID NO. 27) and Pₛₜ-R (downstream primer, GTCCGGCAGGCTCATAGCCCTCACTCCTCCATT, SEQ ID NO. 28). Moreover, the Pₛₜ promoter sequence was obtained through PCR amplification.

Whole gene synthesis was carried out using the P_{aprE} promoter. The primers used were designed as P_{aprE}-F (upstream primer, TATCGATAAGCTTGATATCGCAGCATAATGAACATTTACTCATG, SEQ ID NO. 29) and P_{aprE}-R (downstream primer, GTCCGGCAGGCTCATAGCCCTCACTCCTCCATT, SEQ ID NO. 30). Moreover, the P_{aprE} promoter sequence was obtained through PCR amplification.

The *GNA1* gene that encodes glucosamine 6-phosphate acetylase was obtained from the whole genome of *Saccharomyces cerevisiae* (GenBank No. NM_001179949). Whole gene synthesis with codon optimization was carried out, and an optimized *GNA1* sequence was obtained through PCR amplification. The primers used were designed as gna1-F (upstream primer, GGAGGAGTGAGGGCTATGAGCCTGCCGGACG, SEQ ID NO. 31) and gna1-R (downstream primer, TACAATACCACACATTTTGCGGATCTGCATTTC, SEQ ID NO. 32).

Primers were designed according to the sequence of the *B. licheniformis* MW3 genome (GenBank No. NC_006270.3): glmS-F (upstream primer, ATGCAGATCCGCAAAATGTGTGGTATTGTAGGTTATATTG, SEQ ID NO. 33) and glmS-R (downstream primer, CGGCCGCTCTAGAACTAGTGCTACTCCACCGTCACACTCTT, SEQ ID NO. 34). The *glmS* gene sequence was obtained by PCR amplification using the DNA of the *B. licheniformis* MW3 genome as a template.

Fragments of the three genes, Pₐₗₛ promoter sequence, *GNA1* and *glmS,* collected from PCR amplification were taken, 1 µl for each gene, and inserted into a pHY300PLK vector by seamless cloning to construct a vector pHY300PLK-Pₐₗₛ-GNA1-glmS.

Fragments of the three genes, P₄₃ promoter sequence, *GNA1* and *glmS,* collected from PCR amplification were taken, 1 µl for each gene, and inserted into a pHY300PLK vector by seamless cloning to construct a vector pHY300PLK-P₄₃-GNA1-glmS.

Fragments of the three genes, Pₛₜ promoter sequence, *GNA1* and *glmS,* collected from PCR amplification were taken, 1 µl for each gene, and inserted into a pHY300PLK vector by seamless cloning to construct a vector pHY300PLK-Pₛₜ-GNA1-glmS.

Fragments of the three genes, P_{aprE} promoter sequence, *GNA1* and *glmS,* collected from PCR amplification were taken, 1 µl for each gene, and inserted into a pHY300PLK vector by seamless cloning to construct a vector pHY300PLK-P_{aprE}-GNA1-glmS.

### Example 6: Construction of Engineered Strains BNGS1, BNGS2, BNGS3 and BNGS4

The vector pHY300PLK-Pₐₗₛ-GNA1-glmS was introduced by electroporation into the knockout strain MW3△*nagP*△*gamP*△*gamA*△*nagAB,* resulting in a new *B. licheniformis* strain capable of synthesizing N-acetylglucosamine, named BNGS1.

The vector pHY300PLK-P₄₃-GNA1-glmS was introduced by electroporation into the knockout strain MW3△*nagP*△*gamP*△*gamA*△*nagAB,* resulting in a new *B. licheniformis* strain capable of synthesizing N-acetylglucosamine, named BNGS2.

The vector pHY300PLK-Pₛₜ-GNA1-glmS was introduced by electroporation into the knockout strain MW3△*nagP*△*gamP*△*gamA*△*nagAB,* resulting in a new *B. licheniformis* strain capable of synthesizing N-acetylglucosamine, named BNGS3.

The vector pHY300PLK-P_{aprE}-GNA1-glmS was introduced by electroporation into the knockout strain MW3△*gamP*△*gamA*△*nagAB,* resulting in a new *B. licheniformis* strain capable of synthesizing N-acetylglucosamine, named BNGS4.

### Example 7: Shake Flask Fermentation of Recombinant B. licheniformis BNGS1

### 1. Seed and Fermentation Media

An LB medium (containing10 g/l of sodium chloride, 5 g/l of yeast powder and 10 g/l of peptone) was used as a seed medium. During liquid cultivation, 25 mg/l of tetracycline resistance was added.

A fermentation medium includes following components: 12 g/l of yeast powder; 6 g/l of peptone; 6 g/l of ammonium sulfate; 18.75 g/l of dipotassium hydrogen phosphate trihydrate; 2.5 g/l of potassium dihydrogen phosphate; and 30 g/l of glucose.

### 2. Process of Shake Flask Fermentation

A seed solution containing the BNGS1 strain was inoculated into 5 ml of the LB medium and incubated at 50 °C and 200 rpm for 12-16 h. During cultivation, 25 mg/l of tetracycline resistance was added. It was transferred at an inoculation rate of 5% to a 500-ml Erlenmeyer flask containing 75 ml of the fermentation medium for shake flask fermentation. During cultivation, 25 mg/l of tetracycline resistance was added. The fermentation was run at 40 °C for 50 h, and a 1-ml sample was then taken and centrifuged for 5 min at 12000 rpm. The sample was subjected to filtering by a 0.22-µm aqueous membrane and detection by high-performance liquid chromatography.

### 3. Detection Method

Column: Aminex HPX-87H
Mobile phase: 0.005 mol/L sulfuric acid solution
Flow rate: 0.5 ml/min
Column temperature: 60.0 °C
Injection volume: 20.00 µL
Detector: differential refractive index detector
Detector temperature: 35 °C

The detection results showed that, after 50 hours of shake flask fermentation, N-acetylglucosamine was present at 2.3 g/l in the fermentation broth.

### Example 8: Shake Flask Fermentation of Recombinant B. licheniformis BNGS2

A seed solution containing the BNGS2 strain was inoculated into 5 ml of the LB medium and incubated at 50 °C and 200 rpm for 12-16 h. During cultivation, 25 mg/l of tetracycline resistance was added. It was transferred at an inoculation rate of 5% to a 500-ml Erlenmeyer flask containing 75 ml of the fermentation medium for shake flask fermentation. During cultivation, 25 mg/l of tetracycline resistance was added. The fermentation was run at 40 °C for 50 h, and a 1-ml sample was then taken and centrifuged for 5 min at 12000 rpm. The sample was subjected to filtering by a 0.22-µm aqueous membrane and detection by high-performance liquid chromatography.

The detection results showed that, after 50 hours of shake flask fermentation, N-acetylglucosamine was present at 1.5 g/l in the fermentation broth.

### Example 9: Shake Flask Fermentation of Recombinant B. licheniformis BNGS3

A seed solution containing the BNGS3 strain was inoculated into 5 ml of the LB medium and incubated at 50 °C and 200 rpm for 12-16 h. During cultivation, 25 mg/l of tetracycline resistance was added. It was transferred at an inoculation rate of 5% to a 500-ml Erlenmeyer flask containing 75 ml of the fermentation medium for shake flask fermentation. During cultivation, 25 mg/l of tetracycline resistance was added. The fermentation was run at 40 °C for 50 h, and a 1-ml sample was then taken and centrifuged for 5 min at 12000 rpm. The sample was subjected to filtering by a 0.22-µm aqueous membrane and detection by high-performance liquid chromatography.

The detection results showed that, after 50 hours of shake flask fermentation, N-acetylglucosamine was present at 0.57 g/l in the fermentation broth.

### Example 10: Shake Flask Fermentation of Recombinant B. licheniformis BNGS4

A seed solution containing the BNGS4 strain was inoculated into 5 ml of the LB medium and incubated at 50 °C and 200 rpm for 12-16 h. During cultivation, 25 mg/l of tetracycline resistance was added. It was transferred at an inoculation rate of 5% to a 500-ml Erlenmeyer flask containing 75 ml of the fermentation medium for shake flask fermentation. During cultivation, 25 mg/l of tetracycline resistance was added. The fermentation was run at 40 °C for 50 h, and a 1-ml sample was then taken and centrifuged for 5 min at 12000 rpm. The sample was subjected to filtering by a 0.22-µm aqueous membrane and detection by high-performance liquid chromatography.

The detection results showed that, after 50 hours of shake flask fermentation, N-acetylglucosamine was present at 0.32 g/l in the fermentation broth.

### Example 11: Fed-batch Fermentation of Recombinant B. licheniformis BNGS1 at 37°C in 1-L Fermenter

1. Seed cultivation: a seed solution containing the BNGS1 strain was inoculated into 5 ml of the LB medium and incubated at 50 °C and 200 rpm for 12-16 h. During cultivation, 25 mg/l of tetracycline resistance was added.
2. Shake flask cultivation: it was transferred at an inoculation rate of 5% to 75 ml of the fermentation medium (contained in a 500-ml shake flask). During shake flask cultivation, 25 mg/l of tetracycline resistance was added. The cultivation was conducted at 50 °C and 200 rpm overnight.
3. Fermentation cultivation: the activated seed solution was transferred at an inoculation rate of 4% (v/v) into a 1-L fully automatic fermenter and incubated at a temperature of 37 °C. Tetracycline resistance was added until a working concentration of 25 mg/l was achieved, and a 3mol·l⁻¹hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 70 hours of fermentation, acetylglucosamine was present at 12 g/l in the fermentation broth.

### Example 12: Fed-batch Fermentation of Recombinant B. licheniformis BNGS1 at 50 °C in 1-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 1-L fully automatic fermenter and incubated at a temperature of 50 °C. Tetracycline resistance was added until a working concentration of 25 mg/l was achieved, and a 3mol·l⁻¹hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 70 hours of fermentation, acetylglucosamine was present at 2.8 g/l in the fermentation broth.

### Example 13: Fed-batch Fermentation of Recombinant B. licheniformis BNGS1 at 37 °C in 50-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 50-L fully automatic fermenter and incubated at a temperature of 37 °C. Tetracycline resistance was added until a working concentration of 25 mg/l was achieved, and a 3mol·l⁻¹hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 69.5 hours of fermentation, acetylglucosamine was present at 50.9 g/l in the fermentation broth.

### Example 14: Fed-batch Fermentation of Recombinant B. licheniformis BNGS1 at 37 °C in 50-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 50-L fully automatic fermenter and incubated at a temperature of 37 °C. Tetracycline resistance was added until a working concentration of 25 mg/l was achieved, and a 3mol·l⁻¹hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 73 hours of fermentation, acetylglucosamine was present at 52.5 g/l in the fermentation broth.

### Example 15: Fed-batch Fermentation of Recombinant B. licheniformis BNGS1 at 40 °C in 50-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 50-L fully automatic fermenter and incubated at a temperature of 40 °C. Tetracycline resistance was added until a working concentration of 25 mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 54 hours of fermentation, acetylglucosamine was present at 28.1g/l in the fermentation broth.

### Example 16: Fed-batch Fermentation of Recombinant B. licheniformis BNGS1 at 40 °C in 50-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 50-L fully automatic fermenter and incubated at a temperature of 40 °C. Tetracycline resistance was added until a working concentration of 25 mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 56 hours of fermentation, acetylglucosamine was present at 34.6 g/l in the fermentation broth.

### Example 17: Fed-batch Fermentation of Recombinant B. licheniformis BNGS1 at 45 °C in 50-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 50-L fully automatic fermenter and incubated at a temperature of 45 °C. Tetracycline resistance was added until a working concentration of 25 mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 50 hours of fermentation, acetylglucosamine was present at 24 g/l in the fermentation broth.

### Example 18: Fed-batch Fermentation of Recombinant B. licheniformis BNGS1 at 50 °C in 50-L Fermenter

1. Seed cultivation: the same as Example 11.
2. Shake flask cultivation: the same as Example 11.
3. Fermentation cultivation: the activated seed solution was transferred at an inoculation rate of 4% (v/v) into a 50-L fully automatic fermenter and incubated at a temperature of 50 °C. Tetracycline resistance was added until a working concentration of 25mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 24 hours of fermentation, acetylglucosamine was present at 4.1 g/l in the fermentation broth.

### Example 19: Fed-batch Fermentation of Recombinant B. licheniformis BNGS2 at 37 °C in 50-L Fermenter

1. Seed cultivation: a seed solution containing the BNGS2 strain was inoculated into 5 ml of the LB medium and incubated at 50 °C and 200 rpm for 12-16 h. During cultivation, 25 mg/l of tetracycline resistance was added.
2. Shake flask cultivation: it was transferred at an inoculation rate of 5% to 75 ml of the fermentation medium (contained in a 500-ml shake flask). During shake flask cultivation, 25 mg/l of tetracycline resistance was added. The cultivation was conducted at 50 °C and 200 rpm overnight.
3. Fermentation cultivation: the activated seed solution was transferred at an inoculation rate of 4% (v/v) into a 50-L fully automatic fermenter and incubated at a temperature of 37 °C. Tetracycline resistance was added until a working concentration of 25 mg/l was achieved, and a 3mol·l⁻¹hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 64 hours of fermentation, acetylglucosamine was present at 23.6 g/l in the fermentation broth.

### Example 20: Fed-batch Fermentation of Recombinant B. licheniformis BNGS2 at 45 °C in 50-L Fermenter

1. Seed cultivation: the same as Example 19.
2. Shake flask cultivation: the same as Example 19.
3. Fermentation cultivation: the activated seed solution was transferred at an inoculation rate of 4% (v/v) into a 50-L fully automatic fermenter and incubated at a temperature of 45 °C. Tetracycline resistance was added until a working concentration of 25 mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 49 hours of fermentation, acetylglucosamine was present at 11.6 g/l in the fermentation broth.

### Example 21: Fed-batch Fermentation of Recombinant B. licheniformis BNGS3 at 37 °C in 50-L Fermenter

1. Seed cultivation: a seed solution containing the BNGS3 strain was inoculated into 5 ml of the LB medium and incubated at 50 °C and 200 rpm for 12-16 h. During cultivation, 25 mg/l of tetracycline resistance was added.
2. Shake flask cultivation: it was transferred at an inoculation rate of 5% to 75 ml of the fermentation medium (contained in a 500-ml shake flask). During shake flask cultivation, 25 mg/l of tetracycline resistance was added. The cultivation was conducted at 50 °C and 200 rpm overnight.
3. Fermentation cultivation: the activated seed solution was transferred at an inoculation rate of 4% (v/v) into a 50-L fully automatic fermenter and incubated at a temperature of 37 °C. Tetracycline resistance was added until a working concentration of 25 mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 64 hours of fermentation, acetylglucosamine was present at 10.8 g/l in the fermentation broth.

### Example 22: Fed-batch Fermentation of Recombinant B. licheniformis BNGS3 at 45 °C in 50-L Fermenter

1. Seed cultivation: the same as Example 21.
2. Shake flask cultivation: the same as Example 21.
3. Fermentation cultivation: the activated seed solution was transferred at an inoculation rate of 4% (v/v) into a 50-L fully automatic fermenter and incubated at a temperature of 45 °C. Tetracycline resistance was added until a working concentration of 25 mg/l was achieved, and a 3mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 58 hours of fermentation, acetylglucosamine was present at 7.2g/l in the fermentation broth.

### Example 23: Fed-batch Fermentation of Recombinant B. licheniformis BNGS4 at 37 °C in 50-L Fermenter

1. Seed cultivation: a seed solution containing the BNGS4 strain was inoculated into 5 ml of the LB medium and incubated at 50 °C and 200 rpm for 12-16 h. During cultivation, 25 mg/l of tetracycline resistance was added.
2. Shake flask cultivation: it was transferred at an inoculation rate of 5% to 75 ml of the fermentation medium (contained in a 500-ml shake flask). During shake flask cultivation, 25 mg/l of tetracycline resistance was added. The cultivation was conducted at 50 °C and 200 rpm overnight.
3. Fermentation cultivation: the activated seed solution was transferred at an inoculation rate of 4% (v/v) into a 50-L fully automatic fermenter and incubated at a temperature of 37 °C. Tetracycline resistance was added until a working concentration of 25 mg/l was achieved, and a 3 mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 64 hours of fermentation, acetylglucosamine was present at 6.1 g/l in the fermentation broth.

### Example 24: Fed-batch Fermentation of Recombinant B. licheniformis BNGS4 at 45 °C in 50-L Fermenter

1. Seed cultivation: the same as Example 23.
2. Shake flask cultivation: the same as Example 23.
3. Fermentation cultivation: the activated seed solution was transferred at an inoculation rate of 4% (v/v) into a 50-L fully automatic fermenter and incubated at a temperature of 45 °C. Tetracycline resistance was added until a working concentration of 25 mg/l was achieved, and a 3 mol·l⁻¹ hydrochloric acid solution and a 25% (v/v) ammonia solution were added to adjust and maintain the pH at 7.0. A ventilation rate was kept at 1.5 vvm, and agitation was carried out initially at a speed of 600 r·min⁻¹. Glucose should be continuously controlled at a concentration of 30 g/l. To this end, glucose was supplemented continuously at a constant flow rate upon it being consumed to 3-4 g/l. Cultivation continued until the end of fermentation. A sample was taken therefrom and subjected to detection by high-performance liquid chromatography.

The detection results showed that, after 58 hours of fermentation, acetylglucosamine was present at 2.9 g/l in the fermentation broth.

### Example 25: Stability Assay of BNGS1 Strain

Lines were drawn on a 25 mg/ml solid LB medium plate with a fermentation broth containing a BNGS1 strain. After cultivation for 18 h at 50 °C, 10 single colonies were picked and inoculated into respective 500-ml shake flasks each containing 75 ml of the fermentation medium. The flasks were shaken at a shaker temperature of 40 °C and 200 rpm, and glucose was present at a concentration of 30 g/l. N-acetylglucosamine production stability was assessed using HPLC 70 hours after the inoculation, and the product was determined to be present at a concentration ranging from 2.24 g/l to 2.41 g/l.

Preferred specific embodiments of the present invention have been described in detail above. It is to be understood that, those of ordinary skill in the art can make various modifications and changes based on the concept of the present invention without exerting any creative effort. Accordingly, all the technical solutions that can be obtained by those skilled in the art by logical analysis, inference or limited experimentation in accordance with the concept of the present invention on the basis of the prior art are intended to fall within the protection scope as defined by the claims.

## Claims

1. A genetically engineered strain that produces N-acetylglucosamine, **characterized in that** the genetically engineered strain ferments N-acetylglucosamine under a condition of 40-50°C.

2. The genetically engineered strain according to claim 1, **characterized in that** an original strain of the genetically engineered strain is thermophilus.

3. The genetically engineered strain according to claim 1, **characterized in that** an original strain of the genetically engineered strain is bacillus.

4. The genetically engineered strain according to claim 1, **characterized in that** an original strain of the genetically engineered strain is *Bacillus licheniformis, Bacillus coagulans, Bacillus methylotrophicus,* thermophilic *Bacillus inulinus* or *Geobacillus stearothermophilus.*

5. The genetically engineered strain according to claim 1, **characterized in that** an original strain of the genetically engineered strain is *Bacillus licheniformis* ATCC 14580.

6. The genetically engineered strain according to claim 1, **characterized in that** the genetically engineered strain is *Bacillus licheniformis* BNGS1, deposited in China Center for Type Culture Collection on March 14, 2020 as CCTCC NO: M2020054.

7. The genetically engineered strain according to claim 1, **characterized in that** a catabolism pathway and an intracellular transport pathway of N-acetylglucosamine and N-acetylglucosamine intermediates in an original strain of the genetically engineered strain are blocked.

8. The genetically engineered strain according to claim 7, **characterized in that** the catabolism pathway for N-acetylglucosamine and N-acetylglucosamine intermediates is blocked by deactivation or deletion of one or more of the *nagB* and *gamA* genes for glucosamine 6-phosphate deaminase and the *nagA* gene for N-acetylglucosamine-6-phosphate deacetylase; the intracellular transport pathway for N-acetylglucosamine is blocked by deactivation or deletion of one or both of the *gamP* and *nagP* genes for the N-acetylglucosamine transporter protein.

9. The genetically engineered strain according to claim 7, **characterized in that** over-expressing genes for glucosamine 6-phosphate synthase and glucosamine 6-phosphate acetylase are introduced into the genetically engineered strain.

10. The genetically engineered strain according to claim 8, **characterized in that** a sequence of the gene for glucosamine 6-phosphate acetylase is as shown in SEQ ID NO. 1.

11. The genetically engineered strain according to claim 9, **characterized in that** a sequence of the gene for glucosamine 6-phosphate synthase is as shown in SEQ ID NO. 2.

12. A method of constructing the genetically engineered strain according to any of claims 1 to 11, **characterized in that** the method comprises: deactivating or deleting one or more of the genes for glucosamine 6-phosphate deaminase, N-acetylglucosamine-6-phosphate deacetylase and the N-acetylglucosamine transporter protein in an N-acetylglucosamine catabolism pathway of an original strain; and introducing over-expressing genes for glucosamine 6-phosphate synthase and glucosamine 6-phosphate acetylase.

13. The method according to claim 12, **characterized in** comprising steps of:
A. obtaining a knockout strain by knocking out the *nagB* and *gamA* genes for glucosamine 6-phosphate deaminase, the *nagA* gene for N-acetylglucosamine-6-phosphate deacetylase and the *gamP* and *nagP* genes for the N-acetylglucosamine transporter protein of the original strain;
B. constructing a double-expression vector containing both the *glmS* gene for glucosamine 6-phosphate synthase and the *GNA1* gene for glucosamine 6-phosphate acetylase; and
C. obtaining the genetically engineered strain that produces N-acetylglucosamine by transferring the double-expression vector into the knockout strain obtained in step A.

14. The method according to claim 13, **characterized in that** the double-expression vector is constructed by introducing promoters and expressing the expression vector in series with the promoters and the *glmS* and *GNA1* genes.

15. The method according to claim 14, **characterized in that** the expression vector is pHY300PLK.

16. The method according to claim 14, **characterized in that** the promoters are Pₐₗₛ, P₄₃, Pₛₜ and Pₐₚᵣₑ.

17. The method according to claim 16, **characterized in that** a sequence of the Pₐₗₛ promoter is as shown in SEQ ID NO. 3, a sequence of the P₄₃ promoter is as shown in SEQ ID NO. 4, a sequence of the Pₛₜ promoter is as shown in SEQ ID NO. 5, and a sequence of the Pₐₚᵣₑ promoter is as shown in SEQ ID NO. 6.

18. The method according to claim 13, **characterized in that** the *glmS* gene for glucosamine 6-phosphate synthase originates from *Bacillus licheniformis, Bacillus coagulans, Bacillus methylotrophicus,* thermophilic *Bacillus inulinus* or *Geobacillus stearothermophilus.*

19. The method according to claim 13, **characterized in that** the *GNA1* gene for glucosamine 6-phosphate acetylase originates from *Saccharomyces cerevisiae, Kluyveromyces marxianus* or *Nadsonia fulvescens.*

20. The method according to claim 12 or 13, **characterized in that** the original strain is *Bacillus licheniformis* ATCC 14580.

21. Use of the genetically engineered strain according to any one of claims 1 to 11, **characterized in** being used in production of N-acetylglucosamine.

22. The use according to claim 21, **characterized in that** a fermentation temperature of the production is 40 °C to 50 °C.

23. The use according to claim 21, **characterized in that** a carbon source utilized in the production is glucose, glycerol, xylose or arabinose.

24. The use according to claim 21, **characterized in that** the production comprises steps of:
1) seed cultivation: inoculating the genetically engineered strain into a seed medium, adding tetracycline resistance thereto and culturing it for 12-16 h at 50 °C and 200 rpm for seed activation;
2) shake flask cultivation: transferring the activated seed in step 1) at an inoculation rate of 5% to a shake flask containing a fermentation medium, adding tetracycline resistance thereto and culturing it for 12-16 h at 50 °C and 200 rpm for secondary activation; and
3) fermentation cultivation: transferring secondary activated seed in step 3) to a fermenter at an inoculation rate of 4%, adding tetracycline resistance, adding a carbon source and performing fed-batch fermentation at 40 °C to 50 °C, and culturing it until the end of fermentation.

25. The use according to claim 24, **characterized in that** the seed medium in step 1) comprises following components: peptone, yeast powder and sodium chloride.

26. The use according to claim 24, **characterized in that** the fermentation medium in step 2) comprises following components: yeast powder, peptone, ammonium sulfate, dipotassium hydrogen phosphate trihydrate, potassium dihydrogen phosphate and glucose.

27. The use according to claim 24, **characterized in that** the fermentation medium in step 3) comprises following components: yeast powder, corn steep liquor powder, ammonium sulfate, dipotassium hydrogen phosphate trihydrate, potassium dihydrogen phosphate and glucose.

28. The use according to claim 24, **characterized in that** fermentation conditions in step 3) are: a pH value maintained at 7.0 using a 3 mol/l hydrochloric acid solution and a 25% by volume ammonia solution; a ventilation rate of 1.5 vvm; an initial agitation rate of 600 rpm; a glucose concentration continuously controlled at 30 g/l by supplementing glucose upon it being consumed to 3-4 g/l.
